(19) Europäisches Patentamt
European Patent Office
Office européen des brevets

(11) Numéro de publication: **0 233 101**
**A1**

(12) **DEMANDE DE BREVET EUROPEEN**

(21) Numéro de dépôt: **87400058.1**

(22) Date de dépôt: **13.01.87**

(51) Int. Cl.⁴: **C 07 D 519/04**
A 61 K 31/475

(30) Priorité: **13.01.86 FR 8600364**
**12.12.86 FR 8617412**

(43) Date de publication de la demande:
**19.08.87 Bulletin 87/34**

(84) Etats contractants désignés:
**AT BE CH DE ES FR GB GR IT LI LU NL SE**

(71) Demandeur: **IRE-CELLTARG S.A.**
**Zone Industrielle**
**B-6220 Fleurus (BE)**

(72) Inventeur: **Trouet, André**
**29 Predikherenberg**
**B-3009 Winksele (BE)**

**Dejonghe, Jean-Paul**
**94 rue Ch. Jaumotte**
**B-1350 Wavre (BE)**

**Collard, Marie-Paule**
**18 rue Evenepoel**
**B-1040 Bruxelles (BE)**

**Bhushana, Rao, K.S.P.**
**11 rue Kwakenbienne**
**B-1331 Rosières (BE)**

(74) Mandataire: **Warcoin, Jacques et al**
**Cabinet Régimbeau 26, avenue Kléber**
**F-75116 Paris (FR)**

(54) **Dérivés de vinblastine et composition pharmaceutique les contenant.**

(57) L'invention concerne des dérivés de vinca (indoledihydroindole)-alcaloïde, caractérisés en ce qu'au moins en position $C_3$ et/ou $C_4$ se trouve une chaîne grasse comportant au moins 7 atomes de carbone aliphatique.

Les dérivés selon la présente invention sont utiles comme médicaments.

EP 0 233 101 A1

**Description**

## DERIVES DE VINBLASTINE ET COMPOSITION PHARMACEUTIQUE LES CONTENANT.

La présente invention se rapporte à de nouveaux dérivés de la vinblastine, des procédés pour leur préparation et l'application de ces composés à titre de médicament, notamment comme agent antitumoral.

La vinblastine est un alcaloïde antitumoral connu, isolé à partir de Vinca rosea. La vinblastine (qui sera dénommée ci-après "VLB" en raison de son ancienne DIC : vincaleucoblastine) a été l'objet de nombreuses recherches, études et brevets, en vue de la préparation de substances antitumorales plus actives, plus sélectives et moins toxiques.

La présente invention concerne des dérivés de vinca (indole-dihydroindole)alcaloïde caractérisés en ce qu'au moins en position $C_3$ et/ou $C_4$ se trouve une chaîne grasse comportant au moins 7 atomes de carbone aliphatique.

Parmi les dérivés de vinca alcaloïde selon la présente invention, il faut citer les dérivés en $C_3$ et $C_4$ de la vinblastine, de la vincristine et de la vindésine ; mais d'autres dérivés présentant une activité antinéoplasique peuvent évidemment être envisagés.

Parmi ces dérivés, il faut citer les composés de formule générale I :

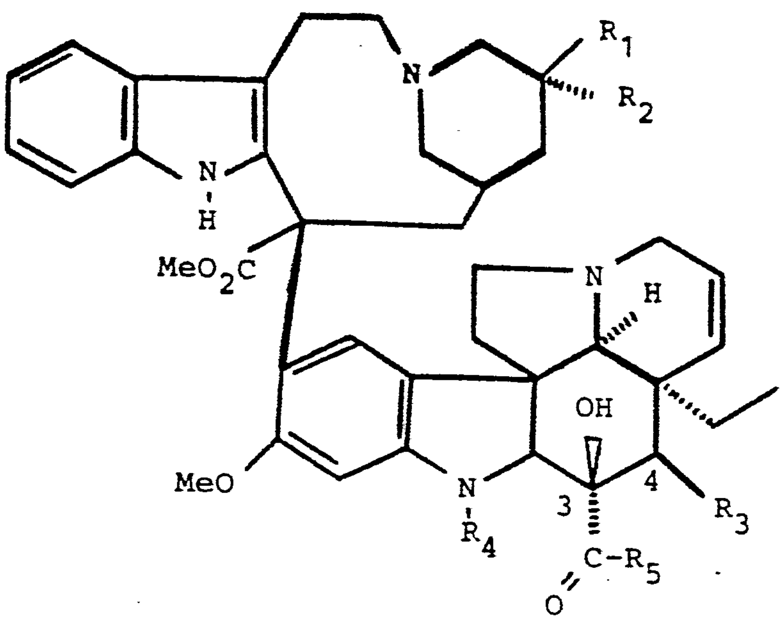

(I)

dans laquelle :

. ($R_1$, $R_2$) représentent (-Et, -OH) ou (-H, -Et),

. $R_4$ représente -H, -Me ou -CHO,

. $R_3$ représente -OH ou -O-C-R'$_3$,
                                   ‖
                                   O

. $R_5$ représente -A-R'$_5$, A étant choisi parmi -NH-, -O-, -NH-alk-C-O, -NH-alk-C-NH,
                                                                 ‖              ‖
                                                                 O              O

. R'$_3$ et R'$_5$ représentent un radical hydrocarboné aliphatique, l'un au moins de R'$_3$ et R'$_5$ ayant au moins 7 atomes de carbone aliphatique, non substitué ou substitué par un ou plusieurs radicaux amino, di($C_1$-$C_3$ alkyl)amino, mono($C_1$-$C_3$ alkyl)amino, NH($C_2$-$C_5$)alcanoyle, cyano, COOH, S-($C_1$-$C_3$ alkyl), COO($C_1$-$C_3$)alkyle, COO-aryle,

. alk désignant une chaîne hydrocarbonée divalente, droite ou ramifiée qui peut comporter un ou plusieurs substituants, ou fonctions, notamment OH, $NH_2$, $CO_2H$, ou bien

. A est le radical divalent d'un amino-acide entrant dans la constitution des protéines se terminant par -NH et -C-O ou -NH et -C-NH, protégé ou non.
  ‖           ‖
  O           O

Parmi les radicaux hydrocarbonée aliphatiques, il faut citer les radicaux :

. alkyle en $C_1$ à $C_{20}$, droits ou ramifiés, et

. alkényle en $C_1$ à $C_{20}$, droits ou ramifiés, ces radicaux étant de préférence droits ; lorsque ces radicaux sont substitués, ils le sont de préférence en bout de chaîne.

Parmi les radicaux alk, il faut citer les radicaux hydrocarbonés, droits ou ramifiés, en $C_1$ à $C_7$, de préférence $C_1$ à $C_5$, non substitués ou substitués par un ou plusieurs radicaux OH, NH ou COOH.

Lorsque A est un radical divalent d'un amino-acide entrant dans la constitution des protéines, il peut être sous forme D ou sous forme L lorsqu'il comporte un atome de carbone asymétrique.

Le radical A peut provenir de Gly, Ala, Val, Leu, Ile, Phe, Ser, Thr, Lys, Lys-OH, Arg, Asp, Asp-$NH_2$, Glu, Glu-$NH_2$ Cys-SH, Met, Tyr, Trp ou His.

Lorsque ces radicaux présentent des fonctions autres que les fonctions de liaison, celles-ci peuvent être protégées par des groupes de protection Pr connus dans la chimie des protéines tels que le groupe Cbz : carbobenzyloxy par exemple.

Le terme aryle désigne de préférence le radicale phényle.

Plus particulièrement, l'invention a pour objet les dérivés répondant à la formule générale (I) dans laquelle :

. $R_1$, $R_2$, $R_3$, $R_4$, $R'_3$ et $R'_5$ ont la même signification que précédemment, mais $R_5$ représente A'-$R''_5$ avec

. A' représentant un radical divalent dérivé d'un acide aminé naturel sous forme D ou L ou d'un dipeptide d'acide aminé naturel sous forme D ou L en particulier dérivé ILE, TRP ou GLY-PHE- se terminant par -NH et

-C ,
‖
O

. $R''_5$ représente -O-$R'_5$ ou NH-$R'_5$.

De préférence, ces dérivés sont tels que A' provient d'un amino-acide choisi parmi le group : ILE, TRP, GLY-PHE et $R'_5$ est un radical hydrocarboné en $C_{10}$ à $C_{16}$.

Dans un mode particulier de l'invention, $R_4$ représente Me et $R_3$ représente OH.

La présente invention concerne également un procédé de préparation des composés de formule générale I, ainsi que les compositions pharmaceutiques comprenant un composé de formule générale I et un excipient, support ou vecteur pharmaceutiquement acceptable.

Les composés de formule générale I selon l'invention peuvent se diviser en deux groupes, selon que la chaîne grasse se trouve en position $C_3$ à $C_4$.

Ainsi, d'une part, les dérivés de vinca alcaloïde pariculièrement préférés sont ceux répondant à la formule I et dans laquelle :

. $R_3$ représente -OH,

. $R_5$ représente -A-'5 avec A choisi parmi : -NH-, -NH-alk-C-O, -NH-alk-C-NH-,

‖ ‖
O O

. $R'_5$ représentant un radical alkyle ou un radical alcényle en $C_7$ à $C_{20}$, non substitué ou substitué par un ou plusieurs radicaux amino, di($C_1$-$C_3$ alkyl)amino, mono($C_1$-$C_3$ alkyl) amino, NH($C_2$-$C_5$)alcanoyle, cyano, COOH, S-($C_1$-$C_3$ alkyl), COO-($C_1$-$C_3$)alkyle, COO-aryle,

. alk désignant une chaîne alcoylène en $C_1$ à $C_3$, ou

. A est un radical divalent dérivant d'un amino-acide entrant dans la constitution de protéines, protégé ou non.

Lorsque A représente -NH-, $R'_5$ est de préférence choise parmi $-(CH_2)_{11}-CH_3$, $-(CH_2)_{17}-CH_3$, $-(CH_2)_{12}-NH_2$ ou $-(CH_2)_7-CH=CH-(CH_2)_8-CH_3$.

Ainsi, par exemple, on appellera composé n° 91 le composé répondant à la formule $I_3$ suivante :

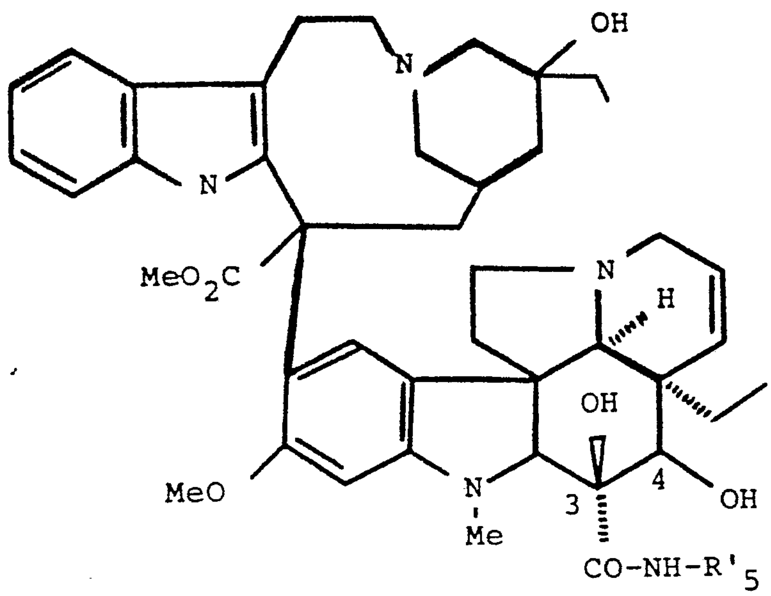

($I_3$)

et dans laquelle $R'_5$ représente $-(CH_2)_{11}-CH_3$.

On appellera composé n° 182 le composé répondant à la même formule $I_3$ et dans laquelle $R'_5$ représente

$-(CH_2)_{12}-NH_2$.

De même, le composé n° 252 sera celui de formule $I_3$ dans laquelle $R'_5$ représente $-(CH_2)_7-CH=CH-(CH_2)_8-CH_3$.

Lorsque A représente un radical divalent dérivé d'un amino-acide entrant dans la constitution des protéines, protégé ou non, alors $R'_5$ est de préférence choisi parmi les radicaux alkyle en $C_7$ à $C_{20}$ et n-alkényle en $C_7$ à $C_{20}$ comportant une ou deux doubles liaisons.

D'autre part, d'autres dérivés de vinca alcaloïde particulièrement préférés selon la présente invention sont ceux répondant à la formule générale I et dans laquelle :

. $R_5$ représente -OMe,

. $R_3$ représente $O-\underset{\underset{O}{\|}}{C}-R'_3$, avec $R'_3$ représentant un radical alkyle ou un radical alkényle en $C_7$ à $C_{20}$, non substitué ou substitué par un ou plusieurs radicaux amino, di($C_1$-$C_3$ alkyl)amino, mono($C_1$-$C_3$ alkyl)amino, NH($C_2$-$C_5$)alcanoyle, cyano, COOH, S-($C_1$-$C_3$ alkyl), COO-($C_1$-$C_3$)alkyle, COO-aryle.

Ainsi, dans la formule $I_4$ suivante :

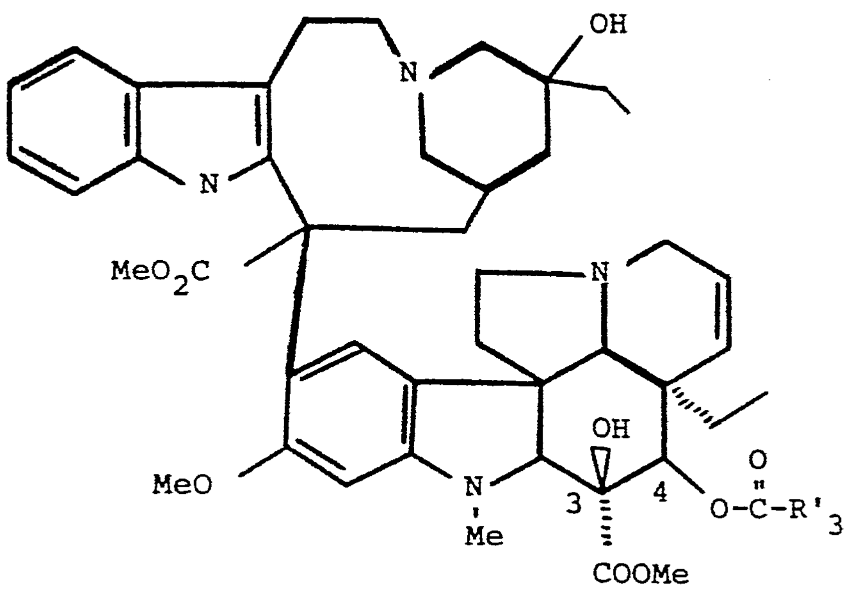

$(I_4)$

. $R'_3$ est de préférence choisi parmi :$-(CH_2)_{14}-CH_3$, $-CH_2CH(COOH)-CH_2-CH=CH-(CH_2)_8-CH_3$, $-CH_2CH(CO-OMe)-CH_2-CH=CH-(CH_2)_8-CH_3$.

La présente invention concerne également des procédés de préparation des composés de formule générale I.

L'invention concerne un procédé de préparation de dérivés d'alcaloïde de vinca en $C_3$, caractérisé en ce que :

a) on fait réagir l'hydrazide de formule :

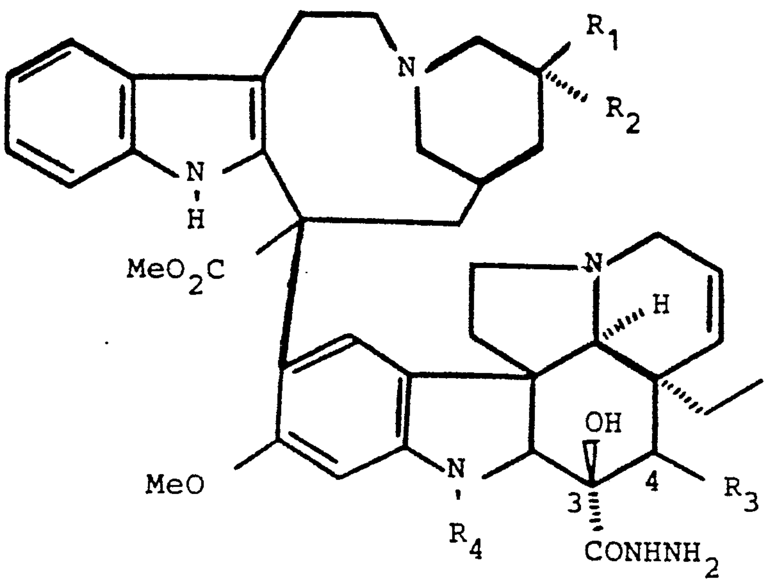

avec un nitrite pour former l'azide correspondant en position $C_3$, et

b) on fait réagir l'azide avec une amine de formule :

H - A - R'$_5$

dans laquelle A et R'$_5$ ont les significations données précédemment, mais A est différent de -O-, pour obtenir le composé désiré

Le schéma réactionnel est alors le suivant :

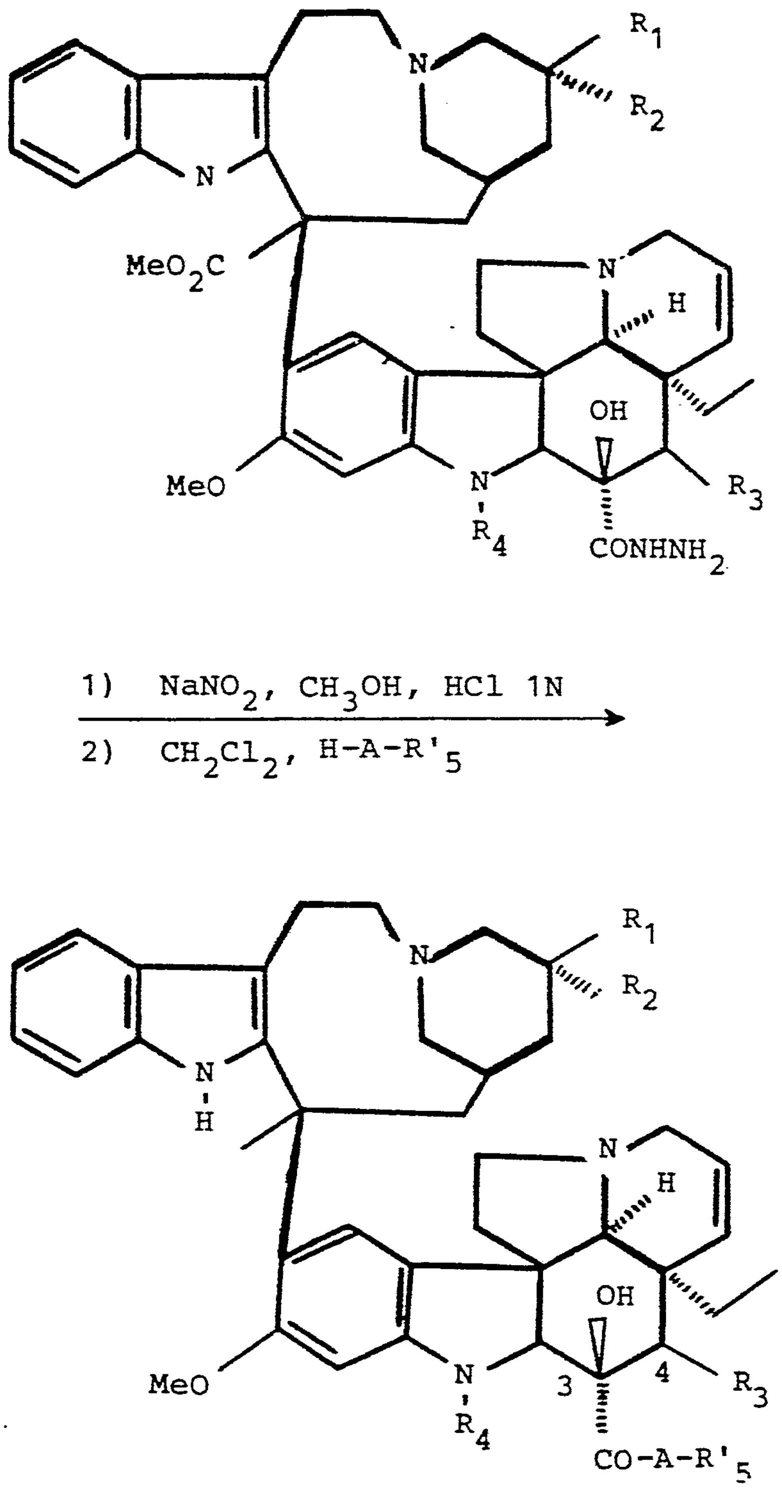


hydrazide de désacétyl-vinblastine

La réaction est effectuée en deux étapes, l'hydrazide est dissous dans un solvant tel que le MeOH en milieu acide, HCl 1 N par exemple, la solution est refroidie et on ajoute un nitrite tel que le nitrite de sodium, le pH est

alors ajusté avec une base faible telle que l'hydrogéno-carbonate de sodium, on obtient ainsi un azide acide qui est extrait par un solvant non miscible tel que le dichlorométhylène.

Après séparation, l'azide, séché et concentré, est traité par l'amine correspondant au produit désiré (H-A-R'$_5$) et le produit brut est séparé du mélange réactionnel et éventuellement purifié.

L'amide obtenu est séparé du milieu réactionnel par tout procédé approprié.

Dans certains cas, la chaîne, latérale peut comporter un atome de carbone asymétrique ; l'invention concerne alors aussi bien le composé sous forme de mélange de stéréoisomères,par exemple le mélange racémique, que les isomères sous forme purifiée.

Selon un mode de réalisation plus particulier, on fait réagir l'azide avec un ester ou un amide de formule H-A'-R"$_5$ dans laquelle A' et R"$_5$ ont les significations données précédemment.

En effet A' se terminant par $\underset{\underset{O}{\|}}{C}$ -, A'-R"$_5$ comprend - $\underset{\underset{O}{\|}}{C}$-O-R'$_5$ ou - $\underset{\underset{O}{\|}}{C}$-NH-R'$_5$ selon que R"$_5$ représente O-R'$_5$ ou NH-R'$_5$ respectivement.

L'invention concerne également l'application des composés de formule I à titre de médicament, ainsi que les compositions pharmaceutique les incorporant et comportant des excipients, supports ou vecteurs pharmaceutiques acceptables.

Les composés selon la présente invention présentent en effet des propriétés antinéoplasiques qui les rendent particulièrement intéressantes dans le traitement des néoplasies malignes telles que les leucémies, la maladie de Hodgkin généralisée, certains lymphomes, les sarcomes ou carcinomes notamment.

Bien entendu, l'activité de ces composés pourra varier en fonction de la structure et les informations données précédemment ne sont qu'indicatives.

Les compositions selon l'invention sont, de préférence, des compositions injectables, en particulier des compositions administrables par voie intraveineuse.

Ces compositions peuvent être réalisées par tout procédé connu dans le domaine des dérivés de vinca, en particulier les produits peuvent être dissous sous forme de sel dans du sérum physiologique contenant éventuellement des excipients donnant un pH correct pour la solution injectable.

Ces compositions peuvent également être conditionnées sous forme de deux conditionnements séparés, lyophilisat de dérivés de VLB et solution physiologique, le mélange étant effectué au moment de l'injection.

Il est également possible de prévoir des formes galéniques plus complexes utilisant le caractère lipophile de ces molécules et mettant à profit d'autres vecteurs pharmaceutiquement acceptables.

Les doses à mettre en oeuvre dépendront du produit, du type de tumeur à traiter et de l'état du malade ; dans le traitement des néoplasies, le posologie est toujours adaptée par le practicien.

Ainsi, il est courant d'utiliser des doses croissantes dans le traitement avec les alcaloïdes de vinca, mais ce type de traitement doit tenir compte de l'évolution d'autres paramètres physiologiques du patient et doit parfois être interrompu.

Dans ces conditions, il n'est pas possible de fournir des dosages à ce stade de la recherche.

Les exemples suivants sont destinés à mettre en évidence d'autres caractéristiques et avantages de la présente invention.

## EXEMPLE 1 :

### Préparation de 4-desacétyl-vinblastine C-3 N-dodécyl carboxamide (composé no 91)

On dissout l'hydrazide de desacétyl-vinblastine (CONRAD et al. J. Med. chem. 22,391 (1979) ) à raison de 0,500 g (0,65 millimole) dans un mélange contenant 10 ml de méthanol et 30 ml de HCl 1 N. La solution est refroidie à 0°C puis est additionnée de 0,103 g de $NaNO_2$ sec (1,49 millimole) en une fois et sous agitation. Après 13 minutes, le pH de la solution est ajusté à 8,5 avec une solution saturée froidie de $NaHCO_3$.

On extrait rapidement l'azide acide de la desacétyl-vinblastine avec 4 x 15 ml de $CH_2Cl_2$, puis on le lave avec une solution saturée de NaCl.

Les extraits sont séchés sur $Na_2SO_4$ et concentrés en un volume de 20 ml. A la solution de $CH_2Cl_2$, on ajoute de la dodecylamine à raison de 0,500 g (2,69) millimoles) et la solution est agitée pendant 2 heures à la température ambiante. La chromatographie en couche mince révèle un composant majeur (Rf = 0,73 ; silice ; méthanol : chlorure de méthylène, 1 : 9 ).

Le solvant est évaporée et le résidu chromatographié sur silice, en éluant d'abord avec 96 :4 puis 93 : 7 de chlorure de méthylène : méthanol. Les fractions appropriées sont combinées et les solvants enlevés pour conduire, à 365 mg de 4-desacétyl-vinblastine C-3 N-dodecyl carboxamide (rendement : 61 %), avec les caractéristiques physiques suivantes :

Rf : 0,73 (silice, $CH_2Cl_2$ : $CH_3OH$, 90 : 10)

SM : (DCI, isobutane) : 922 (M+ + 1), 935 (M+ + 14), 826, 272, 186

RMN : ($CDCl_3$, 360 MHz) : δ 9,50 (mOH), 8,02 (s,1H,NH), 7,51 (d,1H,$H_{9'}$ ), 6,57 (s,1H,$H_9$), 6,05 (s,1H,$H_{12}$), 5,80 (s,2H,$H_{14'}$,$H_{15'}$), 4,16(d,1H,$H_{17}$), 3,95 (m $H_{17}$a'), 3,76 (s,3H,$CH_3O$), 3,59(s,3H,$CH_3O$), 3,36 (s,1H,$H_2$) 2,79(s,3H, $CH_3$-N),2,60(s,1H,$H_{21}$), 1,37-1,20 (m,25 H), 1,02-0,77 ( m,3$CH_3$ + $H_{14'}$)

IR : (KBr, $cm^{-1}$) 3420, 1665, 1730 $cm^{-1}$.

Le sel bis méthane-sufonate est préparé en dissolvant du carboxamide de vinblastine C-3 N-dodecyl (100 mg) dans de l'éthanol anhydre et en ajoutant de l'acide éthanolique méthanesulfonique à 2 %. La solution est

concentrée sous vide. On ajoute au résidu 3 ml d'eau distillée et la solution obtenue est lyophilisée pour donner 123 mg de sel bis-méthanesulfonate.

IR : (KBr) : 3400, 2923, 2858,1729, 1658,1612,1503,1760, 1040 $cm^{-1}$.

EXEMPLE 2 :

Préparation de carboxamide de 4-desacétyl-vinblastine C-3 N-Oleyl (composé n° 252)

Suivant la procédure décrite ci-dessus, 0,500 g d'hydrazide de 4-desacétyl-vinblastine (0,65 millimole) est mis en réaction avec 0,695 g d'oleylamine (2,6 millimole) pour donner le 4-desacétyl-vinblastine C-3 N-oleyl carboxamide (0,310 g, rendement : 47 %) ayant les caractéristiques suivantes :

Rf : 0,72, silice, méthanol : chlorure de méthylène, 1 : 9,

SM (DCI, isobutane), 1005 (M+ + 1)) 922, 810, 733, 502, 392, 279.

IR : (KBr) : 3460, 2920, 2858, 1735, 1663, 1612, 1502, 1460, 1430 $cm^{-1}$

RMN ($CDCl_3$, 360 MHz) : 9,52 (OH), 8.00 (NH), 7,52 (H-9'), 7,17-7,05 (H-10', H-11', H-12'), 6,60(H-9), 6,05 (H-12), 5,81 (H-14',H-15'), 5,35 (-HC=CH-), 4,17(H-17), 3,95 (H-17A'), 3,77 (OMe), 3,58 (OMe), 3,36 (H-2), 2,78(N-Me), 2,60(H-21) 0,97-0,81 (3Me).

Exemple 3 :

Préparation de carboxamide de 4-desacetyl-vinblastine $C_3$ N-octadecyl

Suivant la procédure décrite ci-dessus, on prépare du carboxamide de 4-desacétyl-vinblastine $C_3$-N-ocatdecyl en partant de 0,500 g d'hydrazide de desacétyl-vinblastine (0,65 millimole) et de 0,700 g d'octadecylamine (2,59 millimole). On obtient 0,332 g de carboxamide de 4-desacétyl-vinblastine $C_3$ N-octadécyl (rendement : 51 %).

Ce composé possède les caractéristiques physiques suivantes :

Rf : 0,67 chlorure de méthylène : méthanol, 93 : 7,

IR : (KBr, $cm^{-1}$), 3410, 2920, 2854, 1739, 1663, 1616, 1503, 1460, 1431 $cm^{-1}$

SM : (DCI, $NH_3$), 1007 (M+ + 1), 974, 960, 946

RMN : ($CDCl_3$, 360 MHz) δ : 9,52 (OH), 8,00 (NH), 7,52 (H-9'), 7,17-7,07 (H10', H11', H12'), 6,57 (H-9), 6,05 (H-12), 5,82 (H-14', H-15'), 4,17 (H-17), 3,95 (H-17A'), 3,77 (O-Me), 3,57 (OMe), 3,35 (H-2), 2,80 (N-Me), 2,61 (H-21), 1,37-1,17 (18 $CH_2$), 0,97-0,82 (3 Me).

EXEMPLE 4 :

Préparation de carboxamide de 4-desacétyl-vinblastine $C_3$ N-(12 aminododecyl) (composé n° 182)

Suivant la procédure générale, 0,500 g d'hydrazide de desacétyl-vinblastine (0,65 millimole) sont convertis en 0,327 g de 4 carboxamide de desacétyl-vinblastine C-3 N-(12 aminododecyl) par réaction avec 0,500 g de 1,12-diaminododecane (2,50 millimoles). Le composé est purifié par chromatographie sur alumine, l'élution se faisant avec 92 : 8 de chlorure de méthylène : méthanol. Le composé est isolé en tant que poudre amorphe ayant les caractéristiques suivantes :

Rf : 0,38, alumine, méthanol : chlorure de méthylène 10 : 90

RMN : ($CDCl_3$, 360 MHz) δ : 9,52 ( m, OH), 8, 01 ( m, N-H), 7,51 (d, IH, $H_9$), 6,58 (s, 1H, $H_9$), 6,05 (s, 1H,$H_{12}$), 5,81 (s, 2H, $H_{15'}$, $H_{14'}$) 4,15 (d, 1H, H-17), 3,76 (s, 3H, $CH_3$-O), 3,95 (m, 1H, $H_{17A'}$), 3,59(2,3H,$CH_3$O,3,35 (s,1H,$H_2$), 2,77 (s, 3H, N-$CH_3$), 2,60 (s,1H, $H_{21}$), 2,25 (m, 1H, $H_{17'}$), 1,35-1,24 (m, 23H), 1,00-0,82 ( m, 7H, 2$CH_3$ + $H_{14'}$).

SM$ (DCI (isobutane) 938 (M+ + 1), 965 (M+ + 28), 923 (M+-14), 938, 279.

IR : (KBr) : 3460, 2920, 2853, 1730, 1663, 1605, 1502, 1410, 1225 $cm^{-1}$.

EXEMPLE 5 :

Préparation de 4-palmitoyloxy 4-desacétyl-vinblastine

On dissout 0,500 g de 4-desacétyl-vinblastine (0,65 millimole), et 0,119 g de 4-diméthylaminopyridine (0,975 millimole), dans 10 ml de chlorure de méthylène et la solution est alors refroidie à 0° C. On ajoute 0,214 g de chlorure de palmitoyl (0,78 millimole) en mélangeant.

La chromatographie en couche mince révèle après 24 h la présence d'un composant majeur (silice ; éther : $CH_3OH/NH_3$ 92 : 8). Le solvant est évaporé. La résidu est introduit sur une colonne de silice et élué avec $CH_2Cl_2$ : $CH_3$ OH,93 : 7.

Les fractions appropriées sont collectées, évaporées, fournissant 323 mg de 4-palmitoyloxy 4-desacétyl-vinblastine,(rendement : 49 %).

Caractéristiques physiques :

Rf : 0,65 (silice, $CH_2Cl_2$ : $CH_3OH$ 90 : 10)

IR (KBr) : 3465, 2920, 2855, 1739, 1613, 1500, 1460, 1431, 1362, 1242, 1223 $cm^{-1}$

MS : DIC (isobutane) 1008 (M+ + 1), 990, 958, 884, 756, 586, 512, 413, 256.

RMN : (360 MHz $CDCl_3$) 8,02 (NH), 7,52 (H-9'), 7,17-7,10 (H-10', H-11', H-12') 6,60 (H-9), 6,08 (H-12), 5,82 (H-14'), 5,46 (H-17), 5,28(H-15') 3,97 (H-17 A'), 3,77 (OMe), 3,76 (OMe), 3,68 (H-2), 3,60 (OMe) 2,71 (N-Me),

2,66 (H-21), 1,36-1,21 (16XCH2), 0,93-0,76 (3XCH3).

EXEMPLE 6 :

Préparation de N-(desacétyl-O-4-vinblastinoyl-23)-L-isoleucinate de dodécyl (composé no 860)

Dans les exemples 6 à 12, A′ et R″5 correspondent aux composés du tableau suivant :

| A ' (provenant de) | R"5 | composé n° |
|---|---|---|
| ILE | $-O-(CH_2)_{11}-CH_3$ | 860 |
| ILE | $-HN-(CH_2)_{11}-CH_3$ | 720 |
| ILE | $-HN-(CH_2)_9-CH_3$ | 1370 |
| TRP | $-HN-(CH_2)_9-CH_3$ | 1440 |
| TRP | $-HN-(CH_2)_{11}-CH_3$ | 1470 |
| GLY-PHE | $-HN-(CH_2)_{11}-CH_3$ | 1590 |

On dissout l'hydrazide de desacétyl-vinblastine (CONRAD et al. J. Med. chem. 22,391 (1979)) à raison de 0,500 g (0,65 millimole) dans un mélange contenant 10 ml de méthanol et 30 ml de HCl 1 N. La solution est refroidie à 0°C puis est additionnée de 0,103 g de $NaNO_2$ sec (1,49 millimole) en une fois et sous agitation. Après 13 minutes, le pH de la solution est ajusté à 8,5 avec une solution saturée froide de $NaHCO_3$.

On extrait rapidement l'azide acide de la desacétyl-vinblastine avec 4 x 15 ml de $CH_2Cl_2$, puis on le lave avec une solution saturée de NaCl.

Les extraits sont séchés sur $Na_2SO_4$ et concentrés en un volume de 20 ml. A la solution de $CH_2Cl_2$, on ajoute du dodecyl ester de la L-Isoleucine à raison de 0,500 g et la solution est agitée pendant 2 heures à la température ambiante.

Les solvants sont enlevés et on obtient le N-desacétyl-O-4 vinblastinoyl-23)-L-isoleucinate de dodécyle. Les caractéristiques physiques sont les suivantes :

IR : (KBr, $cm^{-1}$): 3460, 3410, 2958, 2930, 2858, 1737, 1670, 1615, 1502, 1459, 1221, 1010, 740 ;

SM : (DCI, acétone) : 1037 (M+ + 1), 1051 (M+ + 14 + 1) ;

RMN : ($CDCl_3$, 360 MHz):δ 9,36 (OH), 7,97 (NH), 7,46 (H-9′ , (H-10′), 7,10 (H-11′, H-12′), 6,53 (H-12), 6,01 (H-9), 5,81 (H-14), 5,75 (H-15), 4,58 (CH*), 4,17 (H-17, $CH_2$-O), 3,93 (H-17 A′), 3,73 (O-$CH_3$), 3,57 (O-$CH_3$), 3,45 (H-2), 2,86 (H-21 H′), 2,81 (H-21 B′), 2,71 (N-$CH_3$), 2,55 (H-21), 2,42 (H-3 B′), 2,36 (OH-17), 2,02 (H-6 A), 1,91 (H-6 B), 1,25 (12 x $CH_2$), 0,85 - 0,95 (5 x $CH_3$).

EXEMPLE 7 :

Préparation de N-(desacétyl-O-4 vinblastinoyl-23)-L-isoleucinamide de dodecyle (composé 720)

De la même façon, le composé 720 a été préparé à partir d'hydrazide de desacétyl vinblastine et de la dodecylamide de la L-isoleucine.

Ses caractéristiques physiques sont les suivantes :

IR : (KBr, $cm^{-1}$):3460, 2960, 2924, 2855, 1739, 1720, 1658, 1616, 1503, 1460, 1432, 1223, 1009, 740 ;

SM : (DCI, acétone) : 1036 (M+ + 1), 1050 (M+ + 14 + 1), 1064 (M+ + 28 + 1) ;

RMN : ($CDCl_3$, 360 MHz) : δ 9,38 (OH), 7,98 (NH), 7,46 (H-9′, H-10′), 7,10 (H-11′, H-12), 6,54 (H-12), 6,01, (H-9), 5,96 (tr), 5,80 (H-14), 5,75 (H-15), 4,12 (H-17 + m), 3,95 (H-17 A′), 3,76 (O-$CH_3$), 3,58 (O-$CH_3$), 3,47 (H-2), 2,86 (H-21 A′), 2, 83 (H-21-B′), 2,71 (N-$CH_3$), 2,57 (H-21), 2,41 (H-3 B′), 2,01 (H-6A), 1,87 (H-6B), 1,26 (12 x $CH_2$), 0,86 - 0,97 (5 x $CH_3$).

EXEMPLE 8 :

Préparation de N-(désacétyl-O-4 vinblastinoyl-23)-L-isoleucinamide de décyle (composé 1370)

La réaction suivant la procédure de l'exemple 1 entre l'hydrazide de désacetylvinblastine et la décylamide de la L-isoleucine conduit au composé 1370 qui a pour caractéristiques physiques :

IR : (KBr, $cm^{-1}$) : 3464, 2960, 2927, 2880, 2858, 1738, 1720, 1655, 1615, 1502, 1458, 1431, 1221, 738.
SM : (DCI, acétone) : 1008 (M+ + 1), 1022 (M+ + 14 + 1).
RMN : ($CDCl_3$, 360 MHz) : 8,00 (NH), 7,50 (H-9', H-10'), 7,10 (H-11', H-12'), 6,55 (H-12), 6,05 (H-9), 5,93 (tr), 5,81 (H-14), 5,75 (H-15), 4,13 (H-17 + m), 3,96 (H-17 A'), 3,76 ($OCH_3$), 3,60 ($O-CH_3$), 3,45 (H-2), 2,71 ($N-CH_3$), 2,57 (H-21), 2,42 (H-3 B'), 1,26 (10 x $CH_2$), 0,83 -0,95 (5 x $CH_3$).

EXEMPLE 9 :

Préparation de N-(desacétyl-O-4 vinblastinoyl-23)-L-tryptophanamide de dodecyle (composé 1470)

Suivant le procédure décrite pour l'exemple 1, on prépare le composé 1470 en partant d'hydrazide de desacétylvinblastine et de la dodécylamide du L-tryptophane. Ce composé a pour caractéristiques physiques :
IR : (KBr, $cm^{-1}$) : 3400, 2921, 2857, 1737, 1718, 1660, 1606, 1500, 1409, 1224, 1010, 738 ;
SM : (DCI, acétone) : 1109 (M+ + 1), 1123 (M+ + 14 + 1) ;
RMN : ($CDCl_3$, 360 MHz), 8,17 (NH, s), 8,02 (NH,s), 7,80 (1H,d), 7,73 (1H,d), 7,52 (1H,d), 7,35 (1H,d), 7,21-7,02 (m, 5H), 6,59 (H-12), 6,05 (H-9), 5,80 (H-14, H-15), 5,52 (tr, 1H), 4,62 (m, 1H), 4,16 (H-17), 3,96 (H-17 A'), 3,75 (OMe), 3,60 (OMe), 3,48 (H-2), 2,82 H-21 A ! , H-21 B'), 2,72 (N-Me), 2,60 (H-21), 1,12-1,35 (12 x $CH_2$), 0,83-1,02 (3 x $CH_3$).

EXEMPLE 10 :

Préparation de N-(désacétyl-O-4 vinblastinoyl-23)-L-tryptophanamide de decyle (composé 1440)

De la même façon, le composé 1440 a été préparé à partir d'hydrazide de désacetylvinblastine et de la decylamide du L-tryptophane. Ce composé possède les caractéristiques physiques suivantes :
IR : (KBr, $cm^{-1}$): 3400, 2922, 2858, 1720, 1659, 1608, 1500, 1460, 1430, 1225, 1010, 740 ;
SM (DCI, acétone) : 1081 (M+), 1095 (M+ + 14), 1109 (M+ + 28);
RMN ($CDCl_3$, 360 MHz) : 8,20 (NH, s), 8,02 (NH,s) 7,80 (1H,d); 7,73 (1H, d), 750 (1H, d), 7,34 (1H, d), 7,21-7,02 (5H, m), 6,58 (H-12,s), 6,04 (H-9,s), 5,78 (H-14, H-15), 5,52 (tr), 4,61 (m, 1H), 4,17 (H-17), 3,93 (H-17 A'), 3,75 (OMe), 3,60 (OMe), 3,47 (H-2), 2,82 (H-21 A'-H-21 B'), 2,72 (N-Me), 2,60 (H-21), 1,37-1,10 (10 x $CH_2$), 0,80 -1,01 (3 x $CH_3$).

EXEMPLE 11 :

Préparation de N-(désacétyl-O-4 vinblastinoyl-23)-L-glycyl-L-phenylalaninamide de dodecyle (composé 1590)

Suivant la procédure décrite pour l'exemple 1, on prépare le comosé 1590 à partir d'hydrazide de désacétylvinblastine et de la dodécylamide de la L-glycyl-L-phenylalamine. Ses caractéristiques physiques sont les suivantes:
IR : (KBr, $cm^{-1}$) : 3400, 2925, 2857, 1720, 1658, 1615, 1500, 1457, 1227, 740 ;
SM :(DCI, acétone) : 1127 (M+ + 1), 1141 (M+ + 14 + 1) ;
RMN:($CDCl_3$, 360 MHz) : 8, 02 (NH,s), 7,77 (m, 1H), 7,50 (d, 1H), 7,31-7,05 (m, 5H), 6,57 (H-9), 6,07 (H-12), 5,77 (H-14, H-15), 5,62 (tr, 1H), 4,52 (m, 1H), 3,96 (H-17 A'), 4,17 (H-17), 3,76 (OMe), 3,60 (OMe), 3,47 (H-2), 2,85 (H-21 A'-H-21 B'), 2,77 (N-Me), 2,62 (H-21), 1,13 - 1,40 (12 x $CH_2$), 0,85 - 0,98 (3 x $CH_3$).

EXEMPLE 12 :

Activité antitumorale

Des essais d'activité antitumorale des dérivés de la VLB selon la présente invention des exemples 1 à 5 ont été conduits sur des souris de la façon suivante :

12.1. Paramètres d'activité

Deux paramètres d'activité sont utilisés :
- le paramètre "I.L.S." représente l'augmentation de la durée de vie, en pourcentage et selon la relation suivante :

$$I.L.S.\ \% = \frac{T-C}{C} \times 100$$

T (en jours) étant la durée moyenne de vie des souris traitées,
C (en jours) étant la durée moyenne de vie des souris témoins.
- le paramètre "survivants à long terme" dont le calcul en pourcentage est effectué au jour 60.

12.2. Administration intra-veineuse

$10^5$ cellules de leucémie $P_{388}$ sont inoculées au jour 0 à des souris $DBA_2$ femelles, par voie intraveineuse. Le lendemain, le composé 91 selon l'invention est administré par voie intraveineuse.

| Dose (mg/kg/j) | ILS (%) | Survivants jour 60 (%) |
|---|---|---|
| 6,25 | 1 | 0 |
| 12,5 | 1 | 0 |
| 25 | 14 | 0 |
| 50 | 45,9 | 0 |
| 100 | 129 | 0 |
| 150 | 167 | 14 |
| 175 | - 25 | - |
| 200 | - 43 | 0 |

On notera une activité très significative du composé no aux doses de 100 et 150 mg/kg/j.

12.3. Administration intrapéritonéale

$10^6$ cellules de leucémie $P_{388}$ sont inoculées par voie intrapéritonéale chez 6 souris $BDF_1$ femelles au jour 0. Le lendemain, le composé 91 est administré par voie intrapéritonéale.

| Dose (mg/kg/j) | ILS (%) | Survivants jour 60 (%) |
|---|---|---|
| 12,5 | 57 | 0 |
| 25 | 65 | 0 |
| 50 | 77 | 0 |
| 100 | 148 | 0 |
| 150 | - | |
| 200 | 59 (toxique) | 0 |

Comme dans l'exemple12.2, le composé se révèle présenter une activité très significative.

EXEMPLE 13 :

Activité chimiothérapeutique des composés des exemples 6 à 11

Des cellules de leucémie $P_{388}$ sont inoculées à des souris $DBA_2$ femelles, par voie intraveineuse. Le lendemain le composé désigné est administré.

| nombre de cellules p388 | Schéma | Composé n° | Dose (mg/kg/j) | ILS* (%) | Survivants jours 60 |
|---|---|---|---|---|---|
| $10^5$ | i.v./i.v. | 860 | 100 | 131 | 3/10 |
| $10^5$ | i.v./i.v. | 720 | 200 | 57 | 0/10 |
| $10^6$ | ip/ip | | | | |
| $10^6$ | ip/ip | 91 | 150 | 213 | 0/10 |
| $10^6$ | ip/ip | 860 | 50 | 155 | - |
| $10^6$ | ip/ip | 860 | 100 | 172 | - |
| $10^6$ | ip/ip | 860 | 200 | 253 | - |

* ILS représente l'augmentation de la durée de vie, en pourcentage et selon la relation suivante

EMI PA=23 FR=1 HE=15 WI=55 TI=MAT

T (en jours) étant la durée moyenne de vie des souris traitées,
C (en jours) étant la durée moyenne de vie des souris témoins.

Exemple 14 : Influence de la longueur de chaîne sur l'activité et la toxicité.

Le tableau ci-dessous montre un effet très important sur l'activité et la toxicité des dérivés selon l'invention.

On observe que l'augmentation de la longueur de chaîne entraîne :

- une diminution de la toxicité de 10 à 30 fois,
- une augmentation de l'activité,
- des survivants à long terme.

Les produits ont été injectés à leur dose optimale.

Les composés VDS et n° 1610 correspondent à des chaînes courtes.

VDS (vindésines) $R_5 = NH_2$

n° 1610 $R_5 = NH_2\text{-}(CH_2)_5\text{-}CH_3$

Les composés n° 850 et 91 correspondent à des dérivés selon l'invention

n° 850 $R_5 = NH\text{-}(CH_2)_9\text{-}CH_3$

n° 91 $R_5 = NH\text{-}(CH_2)_{11}\text{-}CH_3$

| NOMBRE DE CELLULES P388 | SCHEMA D'INOCU-LATION | SCHEMA DE TRAITE-MENT | PRO-DUIT | DOSE mg/kg | NOMBRE DE SOURIS | ILS % | SUR-VIVANTS (jour 60) |
|---|---|---|---|---|---|---|---|
| $10^5$ | i.v. | i.v. (j.1) | VDS | 4 | 10 | 43 | 0/10 |
| | | | 1610 | 3.13 | 7 | 74 | 0/7 |
| | | | 850 | 50 | 9 | 112 | 2/9 |
| | | | 91 | 150 | 7 | 167 | 1/7 |
| $10^6$ | l.p. | i.p. (j.1) | | | | | |
| | | | VDS | 2.5 | 11 | 150 | 0/11 |
| | | | | 3 | 10 | 89 | 0/10 |
| | | | 91 | 150 | 10 | 213 | 0/10 |

La présence d'une longue chaîne grasse favorise l'activité antitumorale des dérivés de vinca en permettant une synergie entre l'activité lysosomotrope du détergent et l'activité thérapeutique de la vinblastine.

En outre, la présence d'une longue chaîne grasse provoque une augmentation de la lipophile et permet une biodisponibilité différente de ces dérivés vinca, conférant, entre autres, à ces substances médicamenteuse des propriétés, assurant la résorption préférentielle par les canaux lymphatiques de l'intestin grêle aux dépens de la voie sanguine.

**Revendications**

1) Dérivés de vinca (indole-dihydroindole)alcaloïde, caractérisés en ce qu'au moins en position $C_3$ et/ou $C_4$ se trouve une chaîne grasse comportant au moins 7 atomes de carbone aliphatique.

2) Dérivé selon la revendication 1 de formule I :

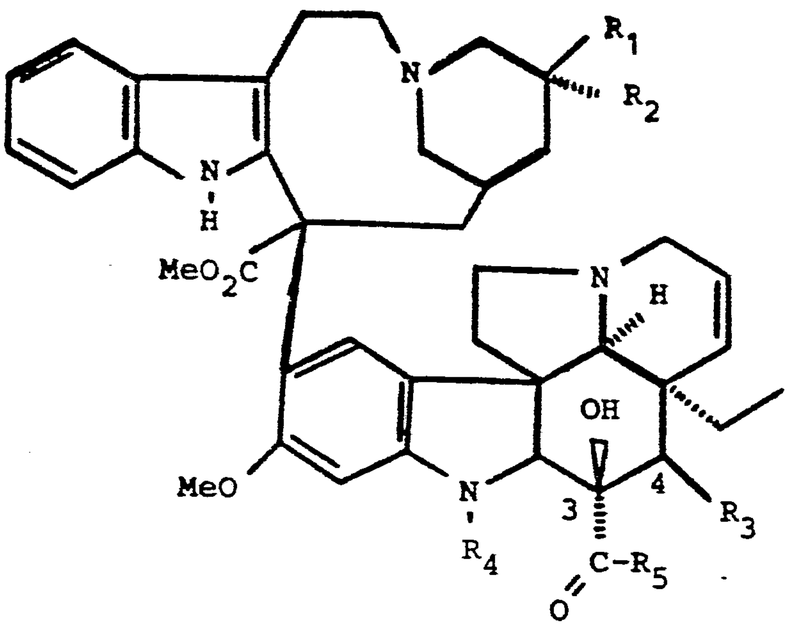

(I)

dans laquelle :

. ($R_1$, $R_2$) représentent (-Et, -OH) ou (-H, -Et)

. $R_4$ représente -H, -Me ou -CHO,

. $R_3$ représente -OH ou $-O-\underset{\underset{O}{\|}}{C}-R'_3$,

. $R_5$ représente $-A-R'_5$, A étant choisi parmi -NH-, -O-, $-NH-alk-\underset{\underset{O}{\|}}{C}-O$, $-NH-alk-\underset{\underset{O}{\|}}{C}-NH$,

. $R'_3$ et $R'_5$ représentent un radical hydrocarboné ayant de 1 à 20 atomes de carbone, l'un au moins des $R'_3$ et $R'_5$ ayant au moins 7 atomes de carbone aliphatique, le radical hydro carboné pouvant être non substitué ou substitué par un ou plusieurs radicaux amino, di($C_1$-$C_3$ alkyl) amino, mono($C_1$-$C_3$ alkyl)amino, NH($C_2$-$C_5$)alcanoyle, cyano, COOH, S-($C_1$-$C_3$ alkyl), COO-($C_1$-$C_3$) alkyl ou COO-aryle,

. alk désignant une chaîne hydrocarbonée divalente, droite ou ramifiée qui peut comporter un ou plusieurs substituants, ou fonctions notamment OH, $NH_2$, $CO_2H$, ou bien

. A est le radical divalent d'un amino-acide entrant dans la constitution des protéines se terminant par -NH- et $-\underset{\underset{O}{\|}}{C}-O-$ ou -NH- et $-\underset{\underset{O}{\|}}{C}-NH$, protégé ou non.

3) Dérivés selon la revendication 2, caractérisés en ce que :

. $R_3$ représente -OH ,

. $R_5$ représente $-A-R'_5$ avec A choisi parmi -NH-, $NH-alk-\underset{\underset{O}{\|}}{C}-O$, $-NH-alk-\underset{\underset{O}{\|}}{C}-NH-$, et

. $R_5$ représentant un radical alkyle ou un radical alkényle en $C_7$ à $C_{20}$, non substitué ou substitué par un ou plusieurs radicaux amino, di($C_1$-$C_3$ alkyl)amino, mono($C_1$-$C_3$ alkyl)amino, NH($C_2$-$C_5$) alcanoyl, cyano, COOH, S-($C_1$-$C_3$ alkyl), COO-aryle,

. Alk désignant une chaîne alcoylène en $C_1$ à $C_3$, ou A est un radical divalent dérivant d'un amino acide entrant dans la constitution des protéines, protégé ou non.

4) Dérivés selon la revendication 3, caractérisés en ce que A représente -NH- et $R'_5$ est choisi parmi $-(CH_2)_{11}-CH_3$, $-(CH_2)_{17}-CH_3$, $-(CH_2)_{12}-NH_2$ et $-(CH_2)_7-CH=CH_2)_8-CH_3$.

5) Dérivés selon la revendication 3, caractérisés en ce que $R'_5$ représente un radical n-alcoyle ou n-alcényle en $C_7$ à $C_{20}$ et A représente un radical divalent dérivé d'un amino acide entrant dans la constitution des protéines protégé ou non.

6) Dérivés selon la revendication 2, caractérisés en ce que dans la formule I :

. $R_5$ représente -OMe,

. $R_3$ représente $-O-\underset{\underset{O}{\|}}{C}-R'_3$, avec $R'_3$ représentant un radical alkyle ou un radical alkényle en $C_7$ à $C_{20}$, non substitué ou substitué par un ou plusieurs radicaux amino, di($C_1$-$C_3$ alkyl)amino, mono($C_1$-$C_3$ alkyl)amino, NH($C_2$-$C_5$)alcanoyl, cyano, COOH S-($C_1$-$C_3$ alkyl), COO-($C_1$-$C_3$)alkyl, COO-aryle.

7) Dérivés selon la revendication 6, caractérisés en ce que R'3 est choisi parmi : $-(CH_2)_{14}-CH_3$, $-CH_2CH(COOH)-CH_2-CH=CH-(CH_2)_8-CH_3$, ou $-CH_2CH(COOMe)-CH_2-CH=CH-(CH_2)_8-CH_3$.

8) Dérivés selon la revendication 1, caractérisés en ce qu'ils répondent à la formule I, dans laquelle :

. ($R_1$, $R_2$) représentent (-Et, -OH) ou (-H, Et)

. $R_4$ représente -H, -Me ou -CHO,

. $R_3$ représente -OH ou $-O-\underset{\underset{O}{\|}}{C}-R'_3$,

. $R_5$ représente $-A'-R''_5$, A' representant un radical divalent dérivé d'un acide aminé naturel sous forme D ou L ou d'un dipeptide d'acide aminé naturel sous forme D ou L en particulier dérivé ILE, TRP ou GLY-PHE, se terminant par -NH et $\underset{\underset{O}{\|}}{C}$-,

. et $R'_3$ et $R'_5$ représentent un radical hydrocarboné ayant de 1 à 20 atomes de carbone, l'un au moins des $R'_3$ et $R'_5$ ayant au moins 7 atomes de carbone aliphatique, le radical hydrocarboné pouvant être non substitué ou substitué par un ou plusieurs radicaux amino, di($C_1$-$C_3$ alkyl) amino, mono ($C_1$-$C_3$ alkyl) amino, $NH(C_2$-$C_5)$alcanoyl, cyano, COOH, S-($C_1$-$C_3$ alkyl), COO-($C_1$-$C_3$) alkyl ou COO-aryle,

. $R''_5$ représente $-O-R'_5$ ou $-NH-R'_5$.

9) Dérivés selon la revendication 8, caractérisés en ce que :

. A' provient d'un amino-acide ou d'un dipeptide d'acide aminé choisi parmi le groupe : ILE, TRP, GLY-PHE, et

. $R'_5$ est un radical hydrocarboné en $C_{10}$ à $C_{16}$.

10) Dérivés selon la revendication 8 ou 9, caractérisés en ce que :

. $R_4$ représente Me et

. $R_3$ représente OH.

11) Dérivés selon l'une des revendications 8 à 10, caractérisés en ce qu'ils sont choisis dans le groupe constitué par les :

N-(désacétyl-O-4 vinblastinoyl-23)-L-isoleucinate de dodécyle ;

N-(désacétyl-O-4 vinblastinoyl-23)-L-isoleucinamide de dodécyle ;

N-(désacétyl-O-4 vinblastinoyl-23)-L-isoleucinamide de décyle ;

N-(désacétyl-O-4 vinblastinoyl-23)-L-tryptophanamide de dodécyle ;

N-(désacétyl-O-4 vinblastinoyl-23)-L-tryptophanamide de décyle ; et

N-(désacétyl-O-4 vinblastinoyl-23)-L-glycyl-L-phénylalaninamide de dodécyle.

12) Procédé de préparation de dérivés d'alcaloïde de vinca en $C_3$, caractérisé en ce que :

a) on fait réagir l'hydrazide de formule :

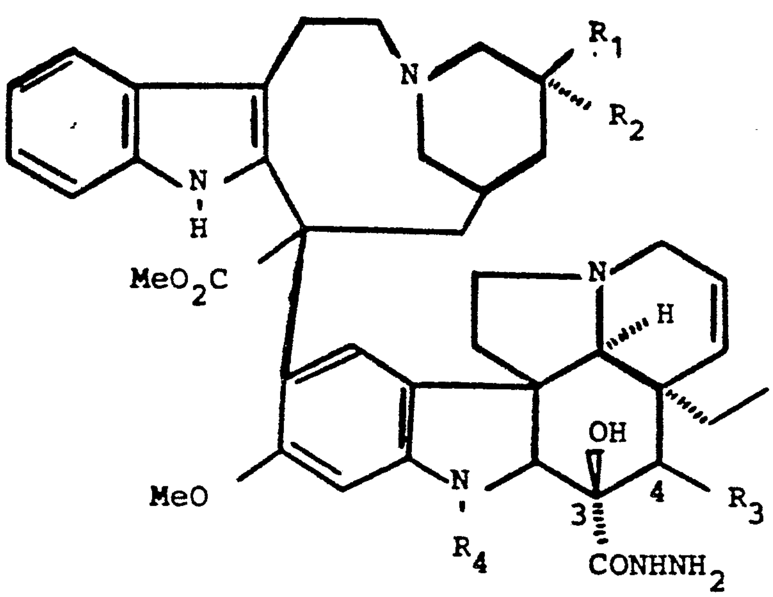

avec un nitrite pour former l'azide correspondant en position $C_3$ et

b) on fait réagir l'azide avec une amine de formule :

$H-A-R'_5$

dans laquelle A et R'5 ont les significations données précédemment mais A est différente de -O-, pour obtenir le composé désiré.

13 ) Procédé selon la revendication 12, caractérisé en ce qu'on fait réagir l'hydrazine de la 4-désacétyl-vinblastine avec la dodécylamine, l'oleylamine, l'octadécylamine ou le 1,12-diamino-dodé-

cane.

14) Procédé de préparation des dérivés d'alcaloïde de vinca en $C_4$ selon la revendication 12, caractérisé en ce qu'on effectue l'éstérification du radical OH en position $C_4$ avec un acide ou un dérivé d'acide réactif de formule :

HO - C - $R'_3$
‖
O

15) Procédé selon la revendication 14, caractérisé en ce qu'on fait réagir la 4-désacétyl-vinblastine avec le chlorure de palmitoyle.

16) Procédé de préparation de dérivés d'alcaloïde de vinca selon la revendication 8, caractérisé en ce que :

a) on fait réagir l'hydrazide de formule :

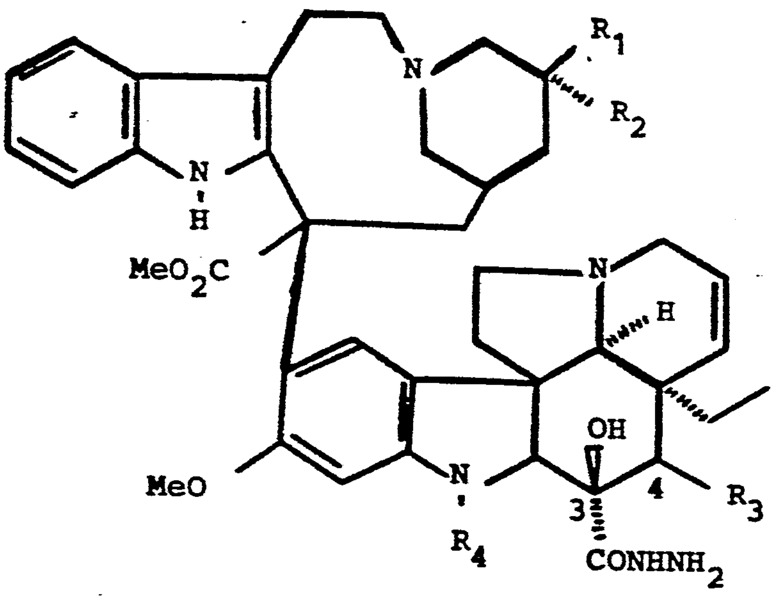

avec un nitrite pour former l'azide correspondant en position $C_3$ et

b) on fait réagir l'azide avec un ester ou un amide de formule $H-A'-R''_5$ dans laquelle A' et $R''_5$ ont les signi fications données précédemment.

17) Procédé selon la revendication 16, caractérisé en ce qu'on fait réagir l'hydrazide de désacétyl-vinblastine avec :

. le dodécylester de la L-isoleucine,
. la dodécylamide de la L-isoleucine,
. la décylamide de la L-isoleucine,
. la dodécylamide du L-tryptophane,
. la décylamide du L-tryptophane,
. la dodécylamide de la L-glycyl-L-phénylalamine.

18) Composition pharmaceutique caractérisé en ce qu'elle comporte un dérivé d'alcaloïde de vinca selon l'une quelconque des revendications 1 à 11, avec un excipient, support ou vecteur pharmaceutiquement acceptable.

19) Composition selon la revendication 18, caractérisée en ce qu'elle est sous forme injectable.

Office européen des brevets

# RAPPORT DE RECHERCHE EUROPEENNE

Numero de la demande

EP 87 40 0058

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-4 203 898 (ELI LILLY AND CO.)<br>* Colonne 21, lignes 30-39; colonne 33, ligne 12 *<br>--- | 1,18 | C 07 D 519/04<br>A 61 K 31/475 |
| A | FR-A-2 400 029 (ELI LILLY AND CO.)<br>* Revendications *<br>----- | 1,18 | |
| | | | DOMAINES TECHNIQUES RECHERCHES (Int. Cl.4) |
| | | | C 07 D 519/00 |

Le présent rapport de recherche a été établi pour toutes les revendications

| Lieu de la recherche | Date d'achèvement de la recherche | Examinateur |
|---|---|---|
| LA HAYE | 14-04-1987 | VAN BIJLEN H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82